# EUROPÄISCHE PATENTSCHRIFT

(11) **EP 3 090 699 B1**
(45) Veröffentlichungstag und Bekanntmachung des Hinweises auf die Patenterteilung: **12.08.2020**
(21) Anmeldenummer: 15166267.3
(22) Anmeldetag: 04.05.2015
(51) Int. Cl.: A61C 1/08, A61C 9/00, A61C 1/16, A61C 1/12, A61B 34/20, A61B 90/00

(54) **SYSTEM FÜR DIE ERFASSUNG UND BEREITSTELLUNG VON DREIDIMENSIONALEN, FÜR DIE KNOCHEN-, WEICHGEWEBS- UND MUNDSITUATION EINES PATIENTEN CHARAKTERISTISCHEN DATEN**
SYSTEM FOR THE COLLECTION AND PROVISION OF THREE-DIMENSIONAL CHARACTERISTIC DATA OF THE BONE, SOFT TISSUE AND MOUTH SITUATION OF A PATIENT
SYSTÈME POUR LA DÉTECTION ET LA FOURNITURE DE DONNÉES CARACTÉRISTIQUES TRIDIMENSIONNELLES DE L'OS, LES TISSUS MOUS ET LA SITUATION BUCCALE D'UN PATIENT

(43) Veröffentlichungstag der Anmeldung: 09.11.2016
(73) Patentinhaber: Weigl, Paul, 60528 Frankfurt am Main (DE); Can, Necati, D:7 Soma (TR)
(72) Erfinder: CAN, Necati, D:7 Soma (TR)
(74) Vertreter: Walkenhorst, Andreas

(56) Entgegenhaltungen:
- DE-A1-102012 003 929
- DE-U1-202010 006 836
- US-A1- 2005 095 554
- US-A1- 2010 305 435
- US-A1- 2012 316 486
- US-A1- 2013 122 463
- US-A1- 2013 244 196

## Beschreibung

Die Erfindung betrifft ein System für die Erfassung und Bereitstellung von dreidimensionalen, für die Knochen-, Weichgewebs- und Mundsituation eines Patienten charakteristischen Daten. Offenbart wird außerdem ein Verfahren zur Erfassung und Bereitstellung solcher Daten.

Bei der Versorgung eines Patienten mit Zahnersatz oder anderen prothetischen Versorgungen können beispielsweise Kronen, Brücken oder andere Versorgungen zum Einsatz kommen. In modernen Behandlungsansätzen können derartige Versorgungen beispielsweise mit Hilfe von Dentalimplantaten in den Patientenmund eingebracht werden. Im Hinblick auf das üblicherweise gewünschte harmonische und optisch und ästhetisch hochwertige Gesamtbild wird die zahntechnische Versorgung oder der jeweilige Zahnersatz dabei individualisiert und angepasst an die jeweilige Mundsituation des Patienten hergestellt.

Um dies zu ermöglichen, müssen zur Vorbereitung einer passgenauen Anfertigung des jeweiligen Zahnersatzes oder auch, bei der Verwendung von Dentalimplantaten, die räumlichen und geometrischen Informationen der Mundsituation des Patienten (beispielsweise Antagonisten, mesial und distal der Insertionsstelle stehende Zähne), der Schleimhaut und insbesondere des für die Stabilität und die Einheilung des Implantats wichtigen Verlaufs und der Gegebenheiten des Kieferknochens erfasst werden. Diese räumlichen und geometrischen Informationen sind insbesondere notwendig, um die Insertion des Implantats passgenau und anatomisch optimiert im Hinblick auf die Eingliederung in die vorhandene Restbezahnung, aber insbesondere auch im Hinblick auf eine stabile Verankerung im Knochen zu ermöglichen. Dabei soll unter anderem berücksichtigt werden, dass beim Einsetzen des Implantats, also bei dem eigentlichen Eindrehen, ausreichend Knochensubstanz im Umfeld der Insertionsstelle vorhanden ist, und dass Perforationen des Kieferknochens zur Seite hin, beispielsweise als Folge einer verkippten oder "schiefen" Einbringung des Implantats, möglichst vermieden werden. Zudem muss die Verletzung von benachbarten anatomischen Strukturen - wie z. B. der n. alveolaris inferior im Unterkiefer - sicher ausgeschlossen werden.

Zu diesem Zweck wird üblicherweise die Mundsituation des Patienten/der Patientin mit einem bildgebenden Verfahren möglichst genau erfasst, um dem Behandler die Möglichkeit zu geben, die Insertion des Implantats insbesondere im Hinblick auf die vorgesehene Insertionsstelle, die in deren Bereich vorhandene Knochensubstanz, den Verlauf der Schleimhaut und dergleichen möglichst genau zu planen. Als bildgebende Verfahren kommen zu diesem Zweck üblicherweise die auf Röntgenstrahlen basierende, zweidimensionale Orthopantomograhie-Technik (OPG), die dreidimensionale 3D-Tomographie-Technik (DVT, CT) oder dergleichen zum Einsatz.

Die zweidimensionalen Röntgendaten können zusätzlich mit Gipsmodellen des Ober- und/oder Unterkiefers kombiniert werden. Die Gipsmodelle entstehen durch den Ausguss von einer so genannten Abformung des Kiefers oder der therapeutisch relevanten Kieferregion. Die Bestimmung der Schleimhautdicke durch das Einstechen von Nadeln bis zur Knochenoberfläche ermöglicht die dritte Dimension - die oro-vestibuläre Dimension - des Kieferkammknochens an der geplanten Insertionsstelle eines Implantats. In der Fachsprache spricht man vom so genannten "Bone-Mapping". Hierzu wird ein rechtwinkliger Querschnitt zum meso-distalen Verlauf des Kieferkammknochens am Gipsmodell produziert und die Schleimhautdicke auf diesen Querschnitt übertragen. Damit die ca. 5-10 Einstichsorte der Nadel ortsgleich auf das Gipsmodell übertragen werden können, werden die Nadelstiche durch eine präfabrizierte, im Mund eingesetzte Schablone geführt. Auf der so ermittelten oro-vestibulären Dimension des Kieferkammknochens basiert die Auswahl des Implantatdurchmessers im Falle eines rotationssymmetrischen Implantats.

Die derzeit mit einer sehr viel höheren Röntgenstrahlenbelastung einher gehende dreidimensionalen bildgebenden Verfahren (z. B. Computertomographie; Digitale Volumentomographie, etc.) als zweidimensionale bildgebende Verfahren enthalten bereits diese Information der oro-vestibulären Dimension des Kieferkammknochens zur Aufnahme eines dentalen Implantates. Diese 3D-Röntgendaten können mit 3D-Aufnahmen von intraoralen optischen Scannern oder mit 3D-Oberflächen-Scans von Gipsmodellen kombiniert (Datenmatching) werden, um eine präzisere und höher aufgelöste Oberflächenrepräsentation von Zähnen und Zahnfleisch zu erhalten.

Anhand der solchermaßen gewonnenen, für die Mundsituation des Patienten charakteristischen Daten, auch als so genannte intraorale Datenabnahme oder intraoraler Scan bezeichnet, kann sodann ein dreidimensionaler digitaler Datensatz erstellt werden, der die Mundsituation des Patienten zumindest im jeweils relevanten Bereich, also in der Umgebung des herzustellenden Zahnersatzes, hinreichend genau wiedergibt. Dieser kann anschließend zur Planung der prothetischen Versorgung und auch der eigentlichen Insertion verwendet werden. Zusätzlich kann auf der Grundlage eines solchen Datensatzes aber auch in der Art einer Echtzeit-Bildgebung der eigentliche Eingriff überwacht werden, wobei dem Behandler beispielsweise anhand einer dreidimensionalen Darstellung der Mundsituation während des Eingriffs angezeigt wird, ob der aktuell verwendete Bohrer oder das Implantat korrekt an der vorher eingeplanten Stelle im Kiefer angesetzt wird, so dass Fehler unmittelbar erkannt und korrigiert werden können.

US2010/0305435 A1 offenbart ein System zur Knochenmarkierung, welches es Chirurgen ermöglicht, computergestützte Behandlungspläne auf den Knochen zu übertragen, wodurch Chirurgen anspruchsvollere Verfahren mit weniger Schwierigkeiten für den Patienten und besseren Ergebnissen durchführen können. Aus US2012/0316486 A1 ist ein System bekannt, welches einen beweglichen Referenzpunkt des Patienten relativ zu einem festen Referenzpunkt kalibrieren und registrieren und diesen Referenzpunkt beibehalten kann, wenn sich der bewegliche Referenzpunkt während eines chirurgischen Eingriffs im Raum bewegt.

Allerdings ist es für einen derartigen Behandlungsansatz, bei dem die gewonnenen Daten unter anderem auch während der eigentlichen Behandlung verfügbar gemacht und zur Verwendung bereitgestellt werden sollen, erforderlich, eine besonders hohe Genauigkeit der erfassten Daten sicherzustellen, um eine zuverlässige Planung der Behandlung zu ermöglichen. Zudem ist es wichtig, dass eine eindeutige Beziehung im Sinne einer Referenzierung zwischen aktuell ermittelten Positionsdaten (beispielsweise für den Bohrer oder ein anderes Behandlungswerkzeug) und dem zuvor ermittelten, in der Art eines digitalen 3D-Modells verwendeten Datensatz sichergestellt ist. Dies ist insbesondere dann problematisch, wenn beispielsweise infolge langer Behandlungsdauern Bewegungen des Patienten nicht ausgeschlossen werden können.

Der Erfindung liegt daher die Aufgabe zu Grunde, ein System für die Erfassung und Bereitstellung von dreidimensionalen, für die Knochen-, Weichgewebs- und Mundsituation eines Patienten charakteristischen Daten anzugeben, mit dem eine besonders hohe Genauigkeit der erfassten Daten auch bei verschiedenen nachfolgenden Verwendungen sichergestellt werden kann. Zudem soll ein hierfür besonders geeignetes Verfahren angegeben werden.

Bezüglich des Systems wird diese Aufgabe erfindungsgemäß gelöst mit den Merkmalen des unabhängigen Anspruchs 1.

Vorteilhafte Ausgestaltungen der Erfindung sind Gegenstand der Unteransprüche. Die Erfindung geht von der Überlegung aus, dass für eine besonders zuverlässige Datenerfassung mit besonders hoher Genauigkeit mögliche Fehlerquellen konsequent vermieden werden sollen. Als Basis für die Datenerfassung ist dabei ein System mit einer Anzahl von Sensoren vorgesehen, deren Position anhand einer Wechselwirkung mit einem von einem Feldgenerator erzeugten Referenzfeld, beispielsweise einem Magnetfeld, bestimmt werden kann. Bei derartigen Systemen ist es üblicherweise erforderlich, in der Art einer Eichung oder Referenzierung einen Fixpunkt festzulegen, in Relation zu dem dann die eigentlichen Positionsdaten, beispielsweise durch Differenzbildung der gemessenen Ortsparameter in den drei Raumkoordinaten gegenüber den jeweiligen Referenz-Ortsparametern, ermittelt werden können.

Um auf einer derartigen Basis die gewünschte hohe Genauigkeit bei der Parameterbestimmung zu ermöglichen, ist nunmehr vorgesehen, möglicherweise entstehende Ungenauigkeiten aufgrund von Mängeln in der Referenzierung konsequent zu vermindern. Dabei soll insbesondere berücksichtigt werden, dass während der Erfassung der Messdaten oder auch nach der eigentlichen Erfassung und vor deren Verwendung, beispielsweise während einer Behandlung, Bewegungen des Patienten so gut wie nicht zu vermeiden sind. Die Referenzierung sollte daher derart erfolgen, dass Fehler aufgrund von Bewegungen durch den Patienten vermieden werden.

Um dies sicherzustellen, ist vorgesehen, den Referenzpunkt oder Bezugspunkt für die Datenerfassung in den Patientenmund hinein zu verlegen. Dadurch resultieren Bewegungen des Patienten während der Erfassung der Messdaten sowohl in einer Verlagerung der jeweils aktuell vermessenen Position als auch in einer entsprechenden Verlagerung des Referenzpunkts, so dass hierdurch entstehende Messwert-Veränderungen bei der anschließenden Auswertung, beispielsweise durch Subtraktion der gemessenen Positionsdaten von den aktuell gemessenen Referenz-Positionsdaten, eliminiert sind.

Um das genannte Konzept zu ermöglichen, umfasst das Datenerfassungssystem einen Feldgenerator zur Erzeugung eines Referenzfeldes, vorzugsweise eines Magnetfeldes, der vorzugsweise außerhalb des Patientenmundes angeordnet, weiterhin bevorzugt aber möglichst nahe am Patientenkopf positioniert ist. Zusätzlich umfasst das System eine Anzahl von Sensoren, die abhängig von ihrer jeweiligen Position im Referenzfeld und ihrer Wechselwirkung mit der lokalen Feldstärke des Referenzfeldes einen Satz von Orts-Parameterdaten liefern. Ein derartiges, als besonders günstig und besonders bevorzugt für die vorliegende Anwendung angesehenes System ist beispielsweise das unter dem Namen Trakstar erhältliche System des Anbieters Ascension Technology Corporation. Dieses System umfasst einen Feldgenerator zur Erzeugung eines Referenzfeldes sowie eine Mehrzahl von Sensoren, die abhängig von ihrer jeweiligen Position im Referenzfeld einen Messdatensatz von 6 Parametern (so genannte "6 degrees of freedom", 6 Freiheitsgrade, d. h. drei Ortskoordinaten und drei Winkelkoordinaten) ausgeben.

Die nunmehr angestrebte besonders hohe Genauigkeit und Zuverlässigkeit bei der Datenerfassung wird auf Basis dieses Systems erreicht, indem einer der Sensoren als Referenzsensor ausgelegt wird, der die Koordinaten des jeweiligen Referenz- oder Bezugspunkts misst und an eine zugeordnete Auswerteeinheit ausgibt. Dieser Referenzsensor ist zur Anbringung innerhalb des Patientenmundes vorgesehen, so dass Bewegungen des Patienten in einer entsprechenden Verschiebung auch des Referenzpunkts resultieren. Um dies zu ermöglichen, ist der Referenzsensor vorliegend auf einem fest im Patientenmund verankerbaren Träger und im Falle eines stattfindenden "bone mappings" und/oder einer anschließend vorgesehenen on-line getrackten Implantatinsertion im jeweils gleichen Kiefer angebracht. Ergänzend hierzu ist ein weiterer der Sensoren als Erfassungs- oder Abtastsensor ausgelegt, mit dem die eigentliche Erfassung von Positions-Messdaten, beispielsweise zur Positionsbestimmung einer Knochengrenze, einer Schleimhautgrenze oder dergleichen, erfolgen soll. Der Abtastsensor ist dazu in der Art einer manuell bedienbaren Ausführung auf einem mit einer Grifffläche versehenen Handgriffel angebracht und zur Erfassung von Mess-Datensätzen über ein manuelles Bedienelement aktivierbar. Damit kann der Benutzer über den Handgriffel den Abtastsensor in eine gewünschte Position bringen, beispielsweise an die Schleimhaut anlegen, und über das Bedienelement die Erfassung der jeweiligen Positions-Messdaten auslösen. Diese werden dann gemeinsam mit den zeitgleich vom Referenzsensor erfassten Referenz-Positionsdaten erfasst, so dass durch Vergleich, beispielsweise durch Subtraktion der jeweiligen Ortskoordinaten, die exakte Lage des aktuell vermessenen Ortes in Relation zum Referenzpunkt ermittelt werden kann.

Auf diese Weise ist es ermöglicht, durch Abtastung einer Vielzahl von Positionen im Mundraum des Patienten die gesamte oder benötigte Mundsituation zu erfassen, ohne dass eine fehlerträchtige Verschiebung des Referenzpunktes beispielsweise in Folge von Bewegungen des Patienten in Kauf genommen werden müsste.

Besonders bevorzugt wird die durch die vorgesehene Anbringung des Referenzsensors im Patientenmund ermöglichte hohe Datengenauigkeit alternativ oder zusätzlich auch zur Bereitstellung von Bilddaten während der Patientenbehandlung in der Art einer Echtzeit-Bildgebung oder eines so genannten "online-Tracking" genutzt. Dazu ist erfindungsgemäß mindestens einer der Sensoren als Werkzeug- oder Bohrer-Überwachungssensor ausgestaltet und auf einem als Träger für einen Zahnarzt-Bohrer vorgesehenen Winkelstück angebracht.

Als "Winkelstück" wird üblicherweise ein Hand- oder Griffstück bezeichnet, an dem ein Zahnarzt-Bohrer oder ein anderes für die Patientenbehandlung vorgesehenes Werkzeug zur Bearbeitung des Kiefers oder Knochens anbringbar ist. Vorteilhafterweise wird ein solcher Werkzeugträger nunmehr ebenfalls mit einem derartigen Sensor bestückt, wobei während der Behandlung des Patienten der ortsfest im Patientenmund angebrachte Referenzsensor dort verbleibt. Die Auswerteeinheit ist dabei in weiterer vorteilhafter Ausgestaltung zur Ermittlung von Bohrer-Positionsdatensätzen durch Vergleich der vom Bohrer-Überwachungssensor erfassten Mess-Datensätze mit einem zeitgleich von dem Referenzsensor erfassten Referenz-Datensatz ausgelegt. Damit kann unter Bezugnahme auf ein und denselben Referenzpunkt, der bereits bei der Erfassung der Mundsituation des Patienten zugrunde gelegt wurde, eine vergleichsweise exakte Positions- und Orientierungsbestimmung des Überwachungssensors am Bohrer vorgenommen werden. Unter der Voraussetzung, dass dieser Überwachungssensor hinreichend starr am Winkelstück oder Werkzeugträger montiert ist, so dass sich seine Lage und Positionierung relativ zum Bohrer oder sonstigen Werkzeug während der Behandlung möglichst nicht ändert, und dass eine hinreichend genaue Eichmessung der Lage und Orientierung des Überwachungssensors relativ zum Bohrer vorgenommen wurde, kann somit in Echtzeit und während der Patientenbehandlung eine Darstellung des Bohrers in Relation zur zuvor erfassten Mundsituation auf einer geeigneten Anzeigeeinheit, beispielsweise einem Bildschirm, erfolgen.

Vorteilhafterweise sind auf der Anzeigeeinheit anhand der Positionsdatensätze erstellte, für die Knochen-, Weichgewebs- und Mundsituation des Patienten charakteristische Konturverläufe darstellbar. Damit kann dem Zahnarzt gerade in Kombination mit der zusätzlichen Darstellung des Bohrers während der Behandlung optisch und unmittelbar die Navigation für den Bohrer zur Verfügung gestellt werden.

In zusätzlicher vorteilhafter Ausgestaltung ist mindestens einer der Sensoren als Kamera-Überwachungssensor ausgestaltet und auf einem Träger für einen Kamera-Aufnahmekopf eines intraoralen Scan-Systems angebracht. Dabei wird dem Umstand Rechnung getragen, dass das vorstehend erläuterte Konzept eines im Patientenmund fest verankerten Referenzsensors auch für den Einsatz in einem an sich bekannten intraoralen 3D-Scansystem zu einer bedeutsamen Verbesserung der Datenqualität und -nutzbarkeit beitragen kann. Derartige Systeme werden in zunehmendem Umfang zur Erstellung von intraoralen Aufnahmen von Zahn- und Kieferkammstrukturen mittels einer intraoralen 3D-Kamera eingesetzt. Insbesondere in der Implantologie werden puderfreie optische 3D-Kameras zunehmend angewendet. Die an sich sehr gute und präzise Oberflächenrepräsentation als 3D-Datensatz im Rechner hat aber bei den bestehenden Systemen keinen zuverlässigen und reproduzierbaren Bezug zum konventionellen "Bone-Mapping" oder für das anschließende On-Line-Tracking des Bohrers für die Realisation einer Knochenkavität zur Insertion eines Implantats. Mit anderen Worten existiert keine Referenz zwischen der 3D-Oberflächenrepräsentation der Kieferkammschleimhaut aus dem intraoralen Scan-Vorgang (z. B. mit einer CEREC-Aufnahmekamera *OmniCam)* und dem Koordinatensystem für das On-Line-Tracking-System.

Dem kann unter Zugrundelegung des nunmehr vorgesehenen Konzepts begegnet werden, indem in vorteilhafter Ausgestaltung einer der Sensoren am Kamera-Aufnahmekopf der Intraoralen Scan-Einheit angebracht wird. Dadurch kann der optische 3D-Datensatz der Kieferkammoberfläche und der Nachbarzähne mit den reellen 3D-Koordinaten des "On-Line-Tracking-Systems" verbunden und in eindeutigen Zusammenhang gebracht werden, so dass die erfassten 3D-Daten des Scans auch für die anschließenden Online-Tracking-Anwendungen nutzbar gemacht werden können. Das hat insbesondere den Vorteil, dass der optische 3D-Datensatz der Kieferkammoberfläche in die Anzeigeeinheit transferiert werden kann. Die dadurch ermöglichte 3D-Darstellung verbessert für den Zahnarzt die Möglichkeiten der Interpretation und der Navigation während der Behandlung erheblich.

Da der Referenzsensor auch bei dieser Anwendung weiterhin an der gleichen Stelle im Mund verbleibt (entweder an einem nicht lockeren Zahn fixiert oder mit einer Nadelkanüle zwischen Schleimhaut und Knochen eingebracht) bleibt auch die Referenzierung erhalten. Mit anderen Worten kann beispielsweise der gleiche Sensor von der optischen Aufnahmeeinheit (Cerec OmniCam) auf das Winkelstück zum Bohren der Knochenkavität rückmontiert werden, oder es wird ein anderer, zusätzlicher Sensor montiert. Aufgrund der Konstanz des Magnetfeldes und der gleichen Position des Referenzsensors in Relation zu den anatomischen Strukturen des Ober- oder Unterkiefers wird dadurch keine weitere Eichung des On-line-Tracking-Systems benötigt, ebenso wie auch nach dem "virtuellen" Bonemapping das anschließend durchgeführte "On-line-Tracking" des Winkelstückes und des Bohrers keine weitere Eichung und Kalibrierung mehr erfordert.

Die ortsgenaue Bestimmung der Schleimhautdicke mit einem derartigen spezifisch ertüchtigten 3D-Datensatz der optischen Kamera vereinfacht sich dementsprechend, da nur noch der Messpunkt auf der Knochenoberfläche des Kieferkamms aktiv mit einer durch die Schleimhaut durchgestochen Nadel gemessen werden muss und nicht mehr zusätzlich auch die Oberfläche der Schleimhaut - diese Schleimhautoberfläche ist ja bereits flächendeckend durch die optische 3D Aufnahme im Messsystem des On-Line-Tracking-Systems vorhanden. Mit anderen Worten ersetzt vorzugsweise die optische 3D Oberflächenmessung der Kieferschleimhautmit einer intraoralen 3D-Kamera die Messung der Schleimhautoberfläche mittels des On-line-Tracking-Verfahrens.

Damit wird die Attraktivität des On-Line-Tracking-Verfahrens durch folgende Vorteile erheblich gesteigert:
1.) Die Messung der Schleimhautdicke wird vereinfacht und zeitlich verkürzt.
2.) Beim On-Line-Tracking des Implantatbohrers besteht eine bessere Orientierung des Zahnarztes wo er sich gerade mit dem Bohrer und dem Winkelstück befindet, da er den Alveolarkamm, die Zähne und die Knochenoberfläche des Kieferkamms virtuell in hoher Qualität dargestellt erkennen kann.
3.) Aufgrund der virtuellen Repräsentation der Nachbarzähne kann die Achsrichtung des Implantats innerhalb der mesio-distalen Ebene besser geplant werden und im System als Vorschlag dargestellt werden. Diese Achse kann dann beim Bohren der Knochenkavität leicht in einem on-line stattfindenden Soll-Ist-Vergleich dem Behandler angezeigt werden. Somit erhält der Behandler stets on-line die Information, ob er die ideale Achsrichtung beim Bohren verlässt oder ob er die Achsrichtung und Position des Implantates einhält.

In besonders vorteilhafter, als eigenständig erfinderisch angesehener Ausgestaltung ist ein Winkelstück im vorstehend genannten Sinne, also insbesondere ein Werkzeugträger für einen Zahnarzt-Bohrer, gezielt zur Verwendung in einem System der vorstehend beschriebenen Art ausgestaltet. Dazu umfasst das Winkelstück vorteilhafterweise einen Handgriff, an dem eine starre Trägerkanüle, vorzugsweise mit einer Länge von mindestens 5 mm, angebracht ist. An deren freiem Ende ist dabei bevorzugt ein zur Verwendung als Bohrer-Überwachungssensor vorgesehener, dem Feldgenerator zugeordneter Sensor angeordnet, dessen Signalleitungen im Innenkanal der Trägerkanüle geführt sind, wobei die Trägerkanüle derart ausgestaltet und positioniert ist, dass der daran angeordnete Sensor mindestens 5 mm beabstandet von dem Handgriff positioniert ist. Durch eine derartige Ausgestaltung ist erreicht, dass eine besonders zuverlässige Positionsdarstellung in Echtzeit im System der vorstehend erläuterten Bauart möglich ist. Durch die annähernd starr ausgeführte Trägerkanüle, deren Innenkanal zur Führung der elektrischen Kontaktleitungen des Sensors vorgesehen ist, so dass diese geschützt und gekapselt vorliegen, ist eine zuverlässige, stabile Positionierung des Sensors relativ zum Bohrer möglich, die sich auch während der Behandlung des Patienten und damit verbundenen Berührungen nicht nennenswert ändert. Damit ist über die gesamte Behandlungsdauer hinweg ein zuverlässiger Rückschluss von der - unmittelbar gemessenen - Position des Sensors auf die - für die Auswertung benötigte und gewünschte - Position des Bohrers möglich.

Durch die vorgesehene Mindestlänge der Trägerkanüle bzw. eine entsprechende Form- oder Geometriegebung ist eine als besonders vorteilhaft angesehene Beabstandung der Position des Sensors, der bevorzugt im End- oder Spitzenbereich der Kanüle angeordnet ist, vom eigentlichen Bohrer gewährleistet. Dies erhöht die Messgenauigkeit, da auf diese Weise vom Bohrer evtl. erzeugte Stör- oder Streufelder keine nennenswerten Auswirkungen auf die im Sensor erfassten Feldparameter haben.

In ganz besonders vorteilhafter, ebenfalls als eigenständig erfinderisch angesehener Ausgestaltung erfolgt die Ertüchtigung des Winkelstücks zur Verwendung in einem System der vorgenannten Art mittels eines dafür vorgesehenen Nachrüstsatzes. Der Nachrüstsatz umfasst dabei einen an einem Grundkörper des Winkelstücks anbringbaren Befestigungsclip, an dem eine starre Trägerkanüle, vorzugsweise mit einer Länge von mindestens 5 mm, angebracht ist, an deren freiem Ende ein zur Verwendung als Bohrer-Überwachungssensor vorgesehener, dem Feldgenerator zugeordneter Sensor angeordnet ist, dessen Signalleitungen im Innenkanal der Trägerkanüle geführt sind, wobei die Trägerkanüle derart ausgestaltet und positioniert ist, dass der daran angeordnete Sensor mindestens 5 mm beabstandet vom Kopf des Winkelstücks positionierbar ist.

Beim Einsatz der genannten Komponenten im Zusammenhang mit einer zahnärztlichen Behandlung ist die sorgfältige Einhaltung der Hygiene- und Sterilitätsvorschriften von ganz besonderer Bedeutung. Insbesondere muss zwingend darauf geachtet werden, dass vor einer Behandlung eines Patienten die verwendeten Instrumente vollständig und zuverlässig gereinigt wurden. Um dies gerade für ein im vorgenannten Sinne für den Einsatz in dem Datenerfassungssystem ertüchtigtes Winkelstück besonders zu vereinfachen, ist in vorteilhafter und ebenfalls als eigenständig erfinderisch angesehener Ausgestaltung eine gezielt an die Trägerkanüle angepasste, als Wegwerfprodukt ausgeführte Schutzkappe, bevorzugt auf Kunststoffbasis, vorgesehen. Diese Schutzkappe weist bevorzugt einen mit seinen Innenabmessungen an die Außenabmessungen der Trägerkanüle angepassten, von einem Kunststoffkörper gebildeten Aufnahmekanal für die Trägerkanüle auf, der an seinem freien Ende in eine zugeordnete Aufnahmeöffnung des Handgriffs bzw. des Befestigungsclips einsteckbar ist. Die Einsteckverbindung für die Schutzkappe im Handgriff bzw. Befestigungsclip ist dabei bevorzugt im Hinblick auf eine besonders hohe Stabilität und Festigkeit, beispielsweise in der Art einer Klemmverbindung, geeignet ausgeführt.

Bezüglich der Verfahrens wird die genannte Aufgabe gelöst, indem ein an einem Handgriffel angeordneter, manuell aktivierbarer Sensor zur Erfassung von Mess-Datensätzen in der Art einer Abtastung an einer zu erfassenden Position im Mundraum des Patienten positioniert und dort aktiviert wird, und bei dem zur Bildung eines für die abgetastete Position charakteristischen Positionsdatensatzes der vom Sensor erfasste Mess-Datensatz in Relation zu einem von einem ortsfest im Mund des Patienten positionierten Referenzsensor erfassten Referenz-Datensatz ausgewertet wird.

Die mit der Erfindung erzielten Vorteile bestehen insbesondere darin, dass durch die vorgesehene Positionierung des Referenzsensors ortsfest im Patientenmund eine von Patientenbewegungen unabhängige und somit besonders fehlerunanfällige Datenerfassung bei der Abtastung der Mundsituation des Patienten ebenso wie während der eigentlichen Behandlung ermöglicht ist. Damit ist auf besonders einfache und kostengünstige Weise in der Art einer Echtzeit-Erfassung oder eines Online-Trackings die Bereitstellung der Daten, ggf. mit zusätzlicher Darstellung des Bohrers oder eines anderen Werkzeugs, auf einer zugeordneten Anzeigeeinheit und somit eine Navigation bei der Führung des Bohrers oder Werkzeugs möglich. Aufgrund des vorgesehenen Messprinzips kann zudem die Datenermittlung unter Verzicht auf für den Patienten belastende Methoden wie beispielsweise Röntgen erfolgen.

Das erfindungsgemäße Konzept der Verankerung des Referenzsensors ortsfest in Bezug zum Patientenmund und zudem innerhalb des Patientenmundes ermöglicht zudem, das konventionelle "Bone mapping" (siehe oben) auf vergleichsweise einfache und zudem sehr kostengünstige Weise zu ersetzen. Insbesondere ist dabei keine Abformung und Herstellung eines Gipsmodells und einer Übertragungsschablone für die Nadelstiche erforderlich. Das Konzept kann zudem das bisherige On-Line-Tracking ersetzen, welches immer auf einen dreidimensionalen Röntgendatensatz (CT, DVT) zurückgreifen musste und dementsprechend kostenaufwendig und mit entsprechenden Strahlenbelastungen für den Patienten verbunden war.

Ein Ausführungsbeispiel der Erfindung wird anhand einer Zeichnung näher erläutert. Darin zeigen:
- FIG. 1: schematisch ein System zur Erfassung und Bereitstellung von dreidimensionalen, für die Knochen-, Weichgewebs- und Mundsituation eines Patienten charakteristischen Daten,
- FIG. 2: die Sensoren des Systems nach FIG. 1 bei einer Verwendung zur Abtastung der Mundsituation des Patienten,
- FIG. 3: die Sensoren des Systems nach FIG. 1 bei einer Verwendung zur Echtzeit-Bildgebung während einer Patientenbehandlung,
- FIG. 4: einen Abtastsensor des Systems nach FIG. 1 in vergrößerter Darstellung, und
- FIG. 5: ein zur Verwendung als Träger für einen Zahnarzt-Bohrer vorgesehenes, mit einem Überwachungssensor des Systems nach FIG. 1 ausgerüstetes Winkelstück.

Gleiche Teile sind in allen Figuren mit denselben Bezugszeichen versehen.

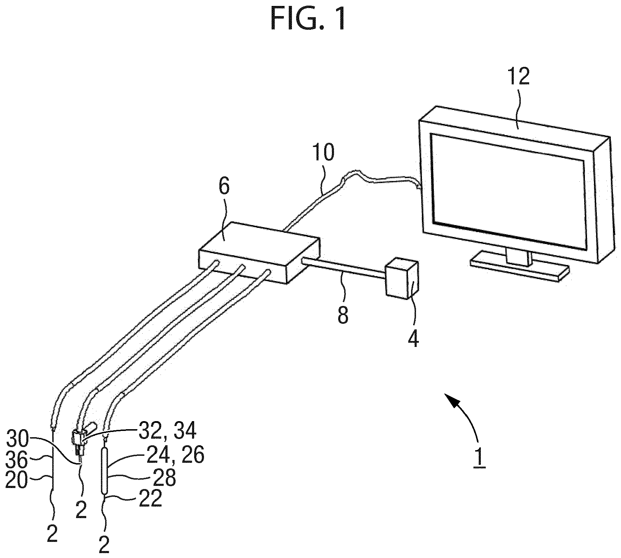

Das System 1 gemäß FIG. 1 ist zum Erfassen und zur Bereitstellung von dreidimensionalen, für die Knochen-, Weichgewebs- und Mundsituation eines Patienten charakteristischen Daten vorgesehen. Zur Ermittlung von digitalen, dreidimensionalen Positionsdatensätzen, mit denen beispielsweise ein digitales dreidimensionales Modell der Mundsituation oder von Teilen davon, beispielsweise eine Darstellung des Kieferknochens des Patienten, erzeugt werden kann, umfasst das System 1 dabei eine Anzahl von Sensoren 2, die mit einem Feldgenerator 4 zusammenwirken. Der Feldgenerator 4 ist dabei zur Erzeugung eines Referenzfeldes, im Ausführungsbeispiel eines Magnetfeldes, ausgelegt, das in einem Raumbereich zumindest in der Umgebung des Feldgenerators 4 räumlich auf charakteristische Weise variiert, so dass das Referenzfeld positionsabhängig spezifische Eigenschaften aufweist. Die Sensoren 2 wiederum sind derart auf das von dem Feldgenerator 4 erzeugte Referenzfeld abgestimmt, dass jeder Sensor 2 abhängig von seiner jeweiligen Position im Referenzfeld und seiner Wechselwirkung mit der lokalen Feldstärke des Referenzfeldes einen Satz von Orts-Parameterdaten liefern. Ein derartiges, als besonders günstig und besonders bevorzugt für die vorliegende Anwendung angesehenes System 1 ist beispielsweise das unter dem Namen Trakstar erhältliche System des Anbieters Ascension Technology Corporation. In dieser Ausgestaltung liefert jeder der Sensoren 2 abhängig von seiner jeweils aktuellen Position im Referenzfeld als auswertbares Ausgabesignal einen Messdatensatz von sechs Parametern (so genannte "6 degrees of freedom", sechs Freiheitsgrade), der aus drei Ortskoordinaten und drei Winkelkoordinaten besteht.

Zur Auswertung der Daten ist eine Auswerteeinheit 6 vorgesehen, an die die Sensoren 2 datenausgangsseitig angeschlossen sind, und die im Ausführungsbeispiel (aber nicht notwendigerweise) auch als Steuereinheit für die Datenerfassung und -ausgabe dient. Dementsprechend ist die Auswerteeinheit 6 auch über eine Signalleitung 8 mit dem Feldgenerator 4 verbunden. Zudem ist die Auswerteeinheit 6 über eine Datenleitung 10 mit einer Anzeigeeinheit 12, insbesondere einem Bildschirm, verbunden, über die die ermittelten Daten oder auch hieraus abgeleitete Informationen graphisch dargestellt werden können.

Bei einem derartigen System 1 liefern die Sensoren 2 üblicherweise Messdaten in Form von Rohdaten, die aus der Wechselwirkung des jeweiligen Sensors 2 mit dem Referenzfeld, beispielsweise einem Magnetfeld, stammen. Um diese Daten in für eine Weiterverarbeitung taugliche Positionsdaten umzuwandeln, gerade wenn dies auch zeitversetzt nachfolgende Anwendungen bereitgestellt werden soll, ist es üblicherweise erforderlich, in der Art einer Eichung oder Referenzierung einen Fixpunkt als Bezugspunkt für die Definition der jeweiligen Positionsdaten festzulegen. Hierzu kann einer der Sensoren 2 als Referenzsensor 20 ausgeführt sein, der als Bezugsbasis für die Ermittlung der Positionsdaten dient. Diese werden dann im Hinblick auf die Relation der von den anderen Sensoren 2 jeweils gelieferten Messdaten ausgewertet, so dass dann deren eigentliche Positionsdaten, beispielsweise durch Differenzbildung der gemessenen Ortsparameter in den drei Raumkoordinaten gegenüber den jeweiligen Referenz-Ortsparametern, ermittelt werden können. Dementsprechend ist einer der Sensoren 2 des Systems 1 als ein derartiger Referenzsensor 20 ausgestaltet.

Ein weiterer der Sensoren 2 ist als Abtastsensor 22 ausgestaltet, der für die Erfassung der dreidimensionalen, für die Knochen-, Weichgewebs- und Mundsituation des Patienten charakteristischen Daten in Kombination mit dem Referenzsensor 20 vorgesehen ist und somit insbesondere zur Verwendung während einer Datenerfassungsphase bereitgehalten wird. Der Abtastsensor 22 ist auf einem mit einer Grifffläche 24 versehenen Handgriffel 26 angebracht und zur Erfassung von Mess-Datensätzen über ein manuelles Bedienelement 28 aktivierbar. Zur Erfassung eines für eine spätere Verarbeitung geeigneten Positionsdatensatzes wird der Abtastsensor 22 mittels des Handgriffels 26 in die zu vermessende Position, beispielsweise eine Oberflächenposition der Schleimhaut oder des Kieferknochens, gebracht und anschließend mittels des Bedienelements 28 aktiviert. Der dieser Position zugeordnete Positionsdatensatz wird anschließend durch Vergleich der vom Abtastsensor 22 erfassten Mess-Datensätze mit dem zeitgleich vom Referenzsensor 20 erfassten Referenz-Datensatz ermittelt. Besonders bevorzugt ist dabei der Referenzsensor jeweils im gleichen Kiefer - also entweder im Unter- oder Oberkiefer - befestigt, an dem auch die Erfassung der anatomischen Strukturen über den Messsensor vorgesehen ist, so dass auch Bewegungen des Unterkiefers gegenüber dem Oberkiefer des Patienten keine Verfälschungen der ermittelten Daten bedingen.

Des Weiteren umfasst das System 1 einen Sensor 2, als Werkzeug- oder Bohrer-Überwachungssensor 30 ausgestaltet und auf einem als Träger 32 für einen Zahnarzt-Bohrer vorgesehenen Winkelstück 34 angebracht. Die Auswerteeinheit 12 ist zur Ermittlung von Bohrer-Positionsdatensätzen durch Vergleich der vom Bohrer-Überwachungssensor 32 erfassten Mess-Datensätze mit einem zeitgleich von dem Referenzsensor 20 erfassten Referenz-Datensatz ausgelegt. Alternativ oder zusätzlich kann zudem mindestens einer der Sensoren 2 als Kamera-Überwachungssensor ausgestaltet und auf einem Träger für einen Kamera-Aufnahmekopf eines nicht näher dargestellten intraoralen Scan-Systems angebracht sein.

Das System 1 ist gezielt für eine besonders hohe Genauigkeit der erfassten Daten auch bei verschiedenen nachfolgenden Verwendungen ausgelegt. Dabei ist insbesondere der Erkenntnis Rechnung getragen, dass die Positionsbestimmung in einem derartigen Erfassungssystem grundsätzlich von einer Bezugnahme auf den Referenzsensor 20 und die von diesem aktuell jeweils gelieferten Messdaten abhängt. Eine zuverlässige und dauerhaft gleichbleibende Positionierung des Referenzsensors 20 ist somit von besonderer Bedeutung für eine zuverlässige Verarbeitung der ermittelten Daten, was bereits durch die gerade bei längeren Behandlungsdauern vermehrt auftretenden Bewegungen des Patienten erschwert wird.

Um dem entgegenzuwirken, ist das System 1 gezielt dafür ausgelegt, den Referenzsensor 20 während der Datenerfassung und ggf. auch während nachfolgender Behandlungsschritte innerhalb des Patientenmundes fest zu verankern und zu positionieren, so dass Bewegungen des Patienten während der Datenerfassung sowohl in einer Positionsänderung des Referenzsensors 20 als auch in einer entsprechenden Positionsänderung des aktuell gerade gemeinsam mit diesem verwendeten Sensor 2 resultieren und sich somit aufgrund der bei der Ermittlung der Positionsdaten angewandten Subtraktion der jeweiligen Datenkomponenten nicht in einer Verfälschung der jeweils ermittelten Daten niederschlagen. Zu diesem Zweck ist der als Referenzsensor 20 ausgestaltete Sensor auf einem fest im Patientenmund verankerbaren Träger 36 angebracht. Die Art der ortsfesten Verankerung im Patientenmund kann dabei abhängig von der jeweiligen Situation des Patienten geeignet gewählt werden. Für den Fall, dass noch eine ausreichende Restbezahnung vorhanden ist, ist es vorteilhaft, den Träger 36 mit dem darauf montierten Referenzsensor 20 an einem belastbaren, ausreichend stabilen Zahn zu befestigen, beispielsweise mittels einer Klemmung oder durch Ankleben oder -zementieren an den jeweiligen Zahn. Für den Fall, dass eine ausreichend stabile Restbezahnung nicht zur Verfügung steht, kann der Referenzsensor 20 aber auch an einer Kanüle oder Injektionsnadel als Träger 36 befestigt sein, die in das Zahnfleisch oder die Schleimhaut eingeschoben wird.

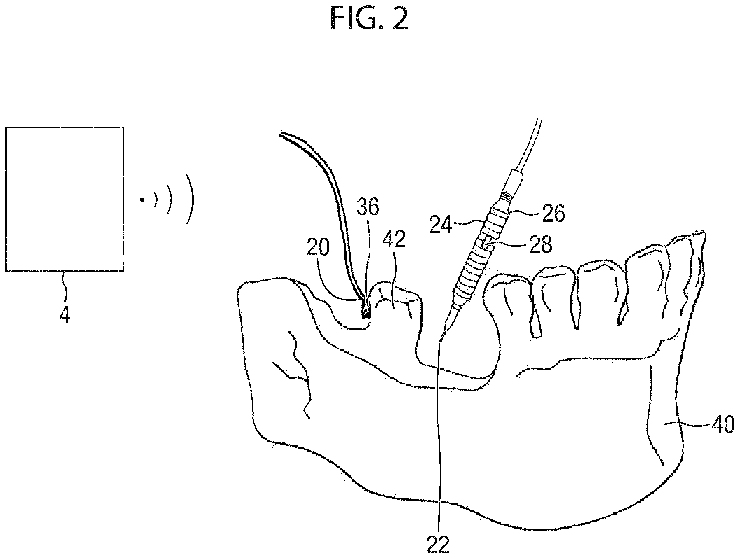

Die Verwendung des Systems 1 und seiner Sensoren 2 bei der Datenerfassung für die Ermittlung der Mundsituation des Patienten ist exemplarisch in FIG. 2 dargestellt, wobei aus Gründen der Vereinfachung lediglich die dabei verwendeten Sensoren 2 und der Kieferknochen 40 des Patienten gezeigt sind. Bei dieser Datenerfassung und auch bei der späteren Überwachung der eigentlichen Behandlung wird der Feldgenerator 4 außerhalb des Patientenmundes, vorzugsweise nah hinter dem Kopf des Patienten, positioniert. Der als Referenzsensor 20 ausgelegte Sensor 2 ist dabei mittels seines Trägers 36 innerhalb des Patientenmundes, im gezeigten Ausführungsbeispiel unmittelbar an einem als ausreichend stabil angesehenen Zahn 42, befestigt, so dass Bewegungen des Patienten in einer entsprechenden Verschiebung auch des Referenzpunkts resultieren und die Qualität der Messergebnisse nicht oder allenfalls geringfügig beeinträchtigen. Im gezeigten Ausführungsbeispiel ist der Träger 36 mit dem Referenzsensor 20 an den Zahn 42 angeklebt und somit fixiert; alternativ könnte aber auch eine mechanische Klemmung am Zahn 42 vorgesehen sein, oder für den Fall, dass kein ausreichend stabiler Zahn zur Fixierung des Trägers 36 zur Verfügung steht, kann der Träger 36 auch als zwischen Zahnfleisch und Kieferknochen 40 einschieb- oder einstechbares Nadelelement oder dergleichen ausgestaltet sein.

Ergänzend hierzu wird ein weiterer der Sensoren 2 als Erfassungs- oder Abtastsensor 22 genutzt, mit dem die eigentliche Erfassung von Positions-Messdaten, beispielsweise zur Positionsbestimmung einer Knochengrenze, einer Schleimhautgrenze oder dergleichen, erfolgt. Hierzu wird der den Abtastsensor 22 tragende Handgriffel 26 geeignet, beispielsweise an der Schleimhaut oder dem Kieferknochen 40 anliegend, positioniert und zur Erfassung des aktuellen Mess-Datensatzes über das manuelle Bedienelement 28 aktiviert. Damit kann der Benutzer somit über das Bedienelement 28 die Erfassung der jeweiligen Positions-Messdaten auslösen. Diese werden dann gemeinsam mit den zeitgleich vom Referenzsensor 20 erfassten Referenz-Positionsdaten erfasst, so dass durch Vergleich, beispielsweise durch Subtraktion der jeweiligen Ortskoordinaten, die exakte Lage des aktuell vermessenen Ortes in Relation zum Referenzpunkt ermittelt werden kann.

Diese Abtastung wird wiederholt für eine Vielzahl von Positionen im Mundraum des Patienten durchgeführt, so dass ausreichend Daten zur Wiedergabe der gesamten oder benötigten Mundsituation erfasst werden. Dies erfolgt, ohne dass eine fehlerträchtige Verschiebung des durch die Positionierung des Referenzsensors 20 gegebenen Referenzpunktes beispielsweise in Folge von Bewegungen des Patienten in Kauf genommen werden müsste.

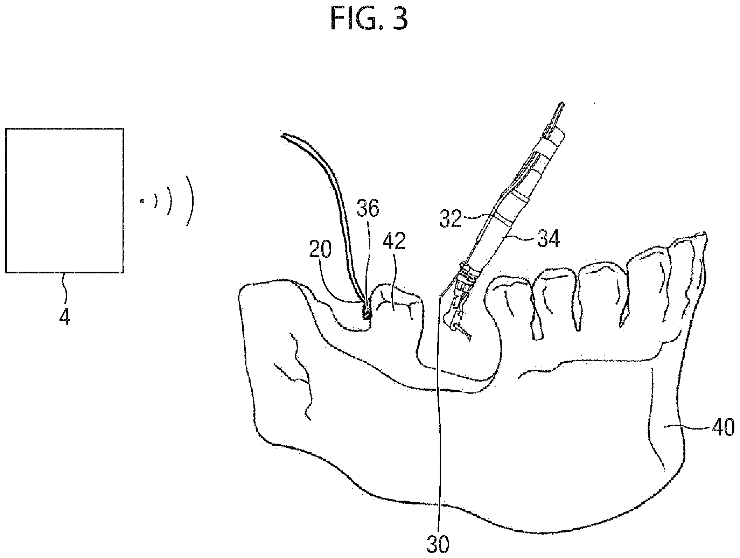

In FIG. 3 ist die Verwendung des Systems 1 und seiner Sensoren 2 während der eigentlichen Behandlung des Patienten exemplarisch dargestellt. Dabei wird die durch die ortsfeste Anbringung des Referenzsensors 20 im Patientenmund ermöglichte hohe Datengenauigkeit zur Bereitstellung von Bilddaten während der Patientenbehandlung in der Art einer Echtzeit-Bildgebung oder eines so genannten "Online-Tracking" genutzt. Dazu wird der als Werkzeug- oder Bohrer-Überwachungssensor 30 ausgestaltete und auf dem als Träger 32 für einen Zahnarzt-Bohrer vorgesehenen Winkelstück 34 angebrachte Sensor 2 in Kombination mit dem Referenzsensor 20 genutzt.

Während der Behandlung des Patienten verbleibt der ortsfest im Patientenmund angebrachte Referenzsensor 20 somit dort. Damit kann unter Bezugnahme auf ein und denselben Referenzpunkt, der bereits bei der Erfassung der Mundsituation des Patienten zugrunde gelegt wurde, eine vergleichsweise exakte Positions- und Orientierungsbestimmung des Überwachungssensors 30 am Bohrer vorgenommen werden. Unter der Voraussetzung, dass dieser Überwachungssensor 30 hinreichend starr am Winkelstück 34 oder Werkzeugträger montiert ist, so dass sich seine Lage und Positionierung relativ zum Bohrer oder sonstigen Werkzeug während der Behandlung möglichst nicht ändert, und dass eine hinreichend genaue Eichmessung der Lage und Orientierung des Überwachungssensors 30 relativ zum Bohrer vorgenommen wurde, kann somit in Echtzeit und während der Patientenbehandlung eine Darstellung des Bohrers in Relation zur zuvor erfassten Mundsituation auf der Anzeigeeinheit 12 erfolgen.

Durch diese Betriebsweise sind auf der Anzeigeeinheit 12 während der Behandlung anhand der Positionsdatensätze erstellte, für die Knochen-, Weichgewebs- und Mundsituation des Patienten charakteristische Konturverläufe darstellbar. Damit kann dem Zahnarzt gerade in Kombination mit der zusätzlichen Darstellung des Bohrers während der Behandlung optisch und unmittelbar die Navigation für den Bohrer zur Verfügung gestellt werden.

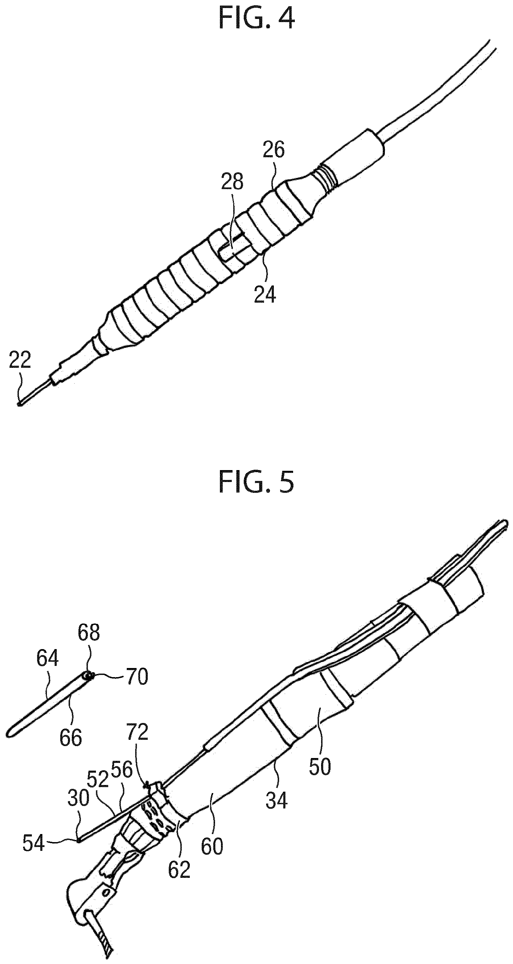

Der Abtastsensor 22 ist, wie dies der vergrößerten Darstellung in FIG. 4 entnehmbar ist, auf dem Handgriffel 26 angeordnet. Dieser umfasst die Grifffläche 24, über die der Bediener die manuelle Positionierung vornehmen kann. An der Grifffläche 24 wiederum ist das manuelle Bedienelement 28 angeordnet, über die der Abtastsensor 22 aktiviert und die Erfassung der Messwerte ausgelöst werden kann.

In FIG. 5 hingegen ist die als eigenständig erfinderisch angesehene Ausgestaltung des für den Einsatz im System 1 ertüchtigten Winkelstücks 34 gezeigt. Als"Winkelstück" wird üblicherweise ein Hand- oder Griffstück bezeichnet, an dem ein Zahnarzt-Bohrer oder ein anderes für die Patientenbehandlung vorgesehenes Werkzeug zur Bearbeitung des Kiefers oder Knochens anbringbar ist. Das gezielt zur Verwendung im System 1 ausgestaltete Winkelstück 34 umfasst einen Handgriff 50, an dem eine Trägerkanüle 52 angebracht ist. An deren freiem Ende 54 ist der Überwachungssensor 30 angeordnet, wobei dessen Signalleitungen 56 im Innenkanal der Trägerkanüle 52 geführt sind. Das Winkelstück 34 ist dabei insbesondere derart ausgestaltet, dass einerseits auch bei variierenden Benutzungssituationen eine zuverlässige, stabile Positionierung des Überwachungssensors 30 relativ zum Bohrer gewährleistet und damit ein zuverlässiger Rückschluss der für den Überwachungssensor 30 jeweils ermittelten Position auf die Position des Bohrers gewährleistet ist, und dass andererseits vom Bohrer evtl. erzeugte Stör- oder Streufelder keine nennenswerten Auswirkungen auf die im Sensor 2 erfassten Feldparameter haben.

Dazu ist die Trägerkanüle 52 als vergleichsweise starres Bauteil aus einem kunststoffbasierten Material mit ausreichender Härte und mit einer Länge von mindestens 5 mm ausgeführt. Durch diese Ausgestaltung ist erreicht, dass eine besonders zuverlässige Positionsdarstellung in Echtzeit in der Anzeigeeinheit 12 des Systems 1 möglich ist.

Die Ertüchtigung des Winkelstücks 34 zur Verwendung im System 1 kann in als eigenständig erfinderisch angesehener Ausgestaltung auch durch Nachrüstung eines bestehenden Winkelstücks 34 erfolgen. Dafür können die erforderlichen Komponenten als Nachrüstsatz bereitgestellt werden. Der Nachrüstsatz umfasst dabei einen Befestigungsclip 62, an dem die Trägerkanüle 52 angebracht ist, und der am Grundkörper 60 des Winkelstücks 34 anbringbar ist.

Beim Einsatz der genannten Komponenten im Zusammenhang mit einer zahnärztlichen Behandlung ist die sorgfältige Einhaltung der Hygiene- und Sterilitätsvorschriften von ganz besonderer Bedeutung. Insbesondere muss zwingend darauf geachtet werden, dass vor einer Behandlung eines Patienten die verwendeten Instrumente vollständig und zuverlässig gereinigt wurden. Um dies für das Winkelstück 34 besonders zu vereinfachen, ist in ebenfalls als eigenständig erfinderisch angesehener Ausgestaltung eine gezielt an die Trägerkanüle 52 angepasste, als Wegwerfprodukt ausgeführte Schutzkappe 64, bevorzugt auf Kunststoffbasis, vorgesehen. Die Schutzkappe 64 weist einen mit seinen Innenabmessungen an die Außenabmessungen der Trägerkanüle 52 angepassten, von einem Kunststoffkörper 66 gebildeten Aufnahmekanal 68 für die Trägerkanüle 52 auf, der an seinem freien Ende 70 in eine zugeordnete Aufnahmeöffnung 72 des Handgriffs 50 bzw. des Befestigungsclips 62 einsteckbar ist. Die Einsteckverbindung für die Schutzkappe 64 im Handgriff 50 bzw. Befestigungsclip 62 ist dabei bevorzugt im Hinblick auf eine besonders hohe Stabilität und Festigkeit, beispielsweise in der Art einer Klemmverbindung, geeignet ausgeführt.

### Bezugszeichenliste

- 1: System
- 2: Sensor
- 4: Feldgenerator
- 6: Auswerteeinheit
- 8: Signalleitung
- 10: Datenleitung
- 12: Anzeigeeinheit
- 20: Referenzsensor
- 22: Abtastsensor
- 24: Grifffläche
- 26: Handgriffel
- 28: Bedienelement
- 30: Überwachungssensor
- 32: Träger
- 34: Winkelstück
- 36: Träger
- 40: Kieferknochen
- 42: Zahn
- 50: Handgriff
- 52: Trägerkanüle
- 54: freies Ende
- 56: Signalleitungen
- 60: Grundkörper
- 62: Befestigungsclip
- 64: Schutzkappe
- 66: Kunststoffkörper
- 68: Aufnahmekanal
- 70: freies Ende
- 72: Aufnahmeöffnung

## Patentansprüche

1. System (1) für die Erfassung und Bereitstellung von dreidimensionalen, für die Knochen-, Weichgewebe- und Mundsituation eines Patienten charakteristischen Daten, mit einem Feldgenerator (4) zur Erzeugung eines Referenzfeldes, das in einem Raumbereich zumindest in der Umgebung des Feldgenerators (4) räumlich auf charakteristische Weise variiert, so dass das Referenzfeld positionsabhängig spezifische Eigenschaften aufweist, und mit einer Anzahl dem Feldgenerator (4) zugeordneter Sensoren (2), die jeweils in Abhängigkeit von an ihrer jeweiligen Position lokal vorliegenden Eigenschaften des vom Feldgenerator (4) erzeugten Referenzfeldes einen für ihre aktuelle räumliche Position charakteristischen Datensatz erzeugen, und die datenseitig mit einer gemeinsamen Auswerteeinheit (6) verbunden sind, wobei mindestens einer der Sensoren (2) als Abtastsensor (22) ausgestaltet, auf einem mit einer Grifffläche (24) versehenen Handgriffel (26) angebracht und zur Erfassung von Mess-Datensätzen über ein manuelles Bedienelement (28) aktivierbar ist, und mindestens einer der Sensoren (2) als Bohrer-Überwachungssensor (30) ausgestaltet und auf einem als Träger (32) für einen Zahnarzt-Bohrer vorgesehenen Winkelstück (34) angebracht ist, und wobei die Auswerteeinheit (6) zur Ermittlung von Positionsdatensätzen durch Vergleich der vom Abtastsensor (22) bzw. vom Bohrer-Überwachungssensor (30) erfassten Mess-Datensätze mit einem jeweils zeitgleich von einem Referenzsensor (20) erfassten Referenz-Datensatz ausgelegt ist, wobei der als Referenzsensor (20) ausgestaltete Sensor (2) auf einem fest im Patientenmund verankerbaren Träger (36) angebracht ist.

2. System (1) nach Anspruch 1, bei dem mindestens einer der Sensoren (2) als Kamera-Überwachungssensor ausgestaltet und auf einem Träger für einen Kamera-Aufnahmekopf eines intraoralen Scan-Systems angebracht ist.

3. System (1) nach Anspruch 1 oder 2, mit einer Anzeigeeinheit (12), auf der anhand der Positionsdatensätze erstellte, für die Knochen-, Weichgewebs- und Mundsituation des Patienten charakteristische Konturverläufe darstellbar sind.

4. Winkelstück (34) zur Verwendung als Träger (32) für einen Zahnarzt-Bohrer in einem System (1) nach einem der Ansprüche 1 bis 3, wobei das Winkelstück (34) einen Handgriff (50) umfasst, an dem eine starre Trägerkanüle (52) angebracht ist, an deren freiem Ende (54) ein zur Verwendung als Bohrer-Überwachungssensor (30) vorgesehener, dem Feldgenerator (4) zugeordneter Sensor (2) angeordnet ist, dessen Signalleitungen (56) im Innenkanal der Trägerkanüle (52) geführt sind, wobei die Trägerkanüle (52) derart ausgestaltet und positioniert ist, dass der daran angeordnete Sensor (2) mindestens 5 mm beabstandet von dem Handgriff (50) positioniert ist.

5. Nachrüstsatz für ein einen Handgriff (50) umfassendes und als Träger (32) für einen Zahnarzt-Bohrer vorgesehenes Winkelstück (34) zu dessen Ertüchtigung zur Verwendung in einem System (1) nach einem der Ansprüche 1bis 3, mit einem an einem Grundkörper (60) des Winkelstücks (34) anbringbaren Befestigungsclip (62), an dem eine starre Trägerkanüle (52) angebracht ist, an deren freiem Ende (54) ein zur Verwendung als Bohrer-Überwachungssensor (30) vorgesehener, dem Feldgenerator (4) zugeordneter Sensor (2) angeordnet ist, dessen Signalleitungen (56) im Innenkanal der Trägerkanüle (52) geführt sind, wobei die Trägerkanüle (52) derart ausgestaltet und positioniert ist, dass der daran angeordnete Sensor (2) mindestens 5 mm beabstandet von dem Handgriff (50) positionierbar ist.

## Claims

1. System (1) for the collection and provision of three-dimensional characteristic data of the bone, soft tissue and mouth situation of a patient, with a field generator (4) for generating a reference field varying in a space zone at least in the vicinity of said field generator (4) regionally in a characteristic way such that said reference field has specific properties depending on the position and with a number of sensors (2) associated to said field generator (4), each generating a data set characteristic for their current position in space, depending on their properties present in their respective positions of the reference field generated by said field generator (4), and connected to a common analysis unit (6) on the data side, at least one of the sensors (2) being configured as sensing sensor (22), mounted on a handgrip (26) equipped with a grip surface (24) and being activatable via a manual control element (28) to collect measurement data sets and at least one of said sensors (2) being configured as drill monitoring sensor (30) and being mounted on an angle piece (34) provided as a carrier (32) for a dentist drill and wherein said analysis unit (6) is configured to determine position data sets by comparison of measurement data sets collected by said sensing sensor (22) respectively by said drill monitoring sensor (30) with a reference data set collected simultaneously by a reference sensor (20), said sensor (2) configured as a reference sensor (20) being firmly mounted in the carrier (36) anchorable in the patient's mouth.

2. The system (1) according to claim 1, where at least one of the sensors (2) is configured as camera monitoring sensor and is mounted on a carrier for a camera reception head of an intraoral scan system.

3. The system (1) according to claim 1 or 2 having a display unit (12) where characteristic contour courses established by means of position data sets for the patient's bone, soft tissue and mouth situation are representable.

4. An angle piece (34) to be used as a carrier (32) for a dentist drill in a system (1) according to one of the claims 1 to 3, said angle piece (34) comprising a handgrip (50), where a rigid carrier hollow needle (52) is mounted on, at the free end (54) of which, a sensor (2) associated to said field generator (4) and provided to be used as drill monitoring sensor (30) and the signal conducts (56) of which are guided in the inner channel of said carrier hollow needle (52), said carrier hollow needle (52) being configured and positioned such that said sensor (2) mounted thereon is positioned from said handgrip (50) in a distance of at least 5 mm.

5. A backfitting set for an angle piece (34), comprising a handgrip (50) and provided as a carrier (32) for a dentist drill designed for its strengthening and to be used in a system (1) according to one of the claims 1 to 3, having an attachment clip (62) mountable to a base body (60) of sad angle piece (34), where a rigid carrier hollow needle (52) is mounted thereon, at the free end (54) of which a sensor (2) associated to said field generator (4) and provided to be used as drill monitoring sensor (30) and the signal conducts (56) of which are guided in the inner channel of said carrier hollow needle (52), said carrier hollow needle (52) being configured and positioned such that said sensor (2) mounted thereon is positionable from said handgrip (50) in a distance of at least 5 mm.

## Revendications

1. Système (1) pour la détection et la fourniture de données caractéristiques tridimensionnelles de l'os, des tissus mous et la situation buccale d'un patient avec un générateur de champ (4) pour générer un champ de référence qui varie dans une zone spatiale au moins aux environs du générateur de champ (4) de manière spatiale et caractéristique de sorte que le champ de référence présente des propriétés spécifiques en fonction de la position, et avec un nombre de capteurs associés (2) au générateur du champ (4) qui génèrent chacun un paquet caractéristique pour leur position spatiale actuelle respective en fonction des propriétés localement présentes du champ de référence généré par le générateur de champ (4) et qui sont liés à une unité d'analyse (6) commune au niveau des données, au moins l'un des capteurs (2) étant configuré comme capteur à balayage (22), monté sur une poignée (26) munie d'une surface de poignée (24) et étant activable pour la détection de paquets de mesures via un élément de commande (28) manuel et au moins un des capteurs (2) est configuré comme capteur de surveillance (30) de fraise et est monté sur une pièce coudée (34) prévue comme support (32) pour une fraise de dentiste et où l'unité d'analyse (6) est configurée pour déterminer des paquets de position moyennant la comparaison des paquets de mesure captés par le capteur à balayage (22) voire le capteur de surveillance (30) de fraise avec un paquet de référence capté à chaque fois en même temps par un capteur de référence (20), le capteur (2) configuré comme un capteur de référence (20) étant monté sur un support (36) solidarisé sur la bouche d'un patient.

2. Système (1) selon la revendication 1, où au moins l'un des capteurs (2) est configuré comme un capteur de surveillance de caméra et est monté sur un support pour une tête de réception de caméra d'un système de scan intraoral.

3. Système (1) selon la revendication 1 ou 2 ayant une unité d'affichage (12) à laquelle des contours caractéristiques du patient pour l'os, les tissus mous et la situation buccale d'un patient établis à l'aide des paquets de position sont représentables.

4. Pièce angulaire (34) à utiliser comme support (32) pour une fraise de dentiste dans un système (1) selon l'une des revendications 1 à 3, la pièce angulaire (34) comprenant une manette (50) à laquelle est fixée une canule de support (52) rigide à l'extrémité libre (54) de laquelle est disposé un capteur (2) associé au générateur de champ (4) et prévu pour une utilisation comme capteur de surveillance (30) de fraise et dont les conduites de signal (56) sont guidées à l'intérieur de la canule de support (52), la canule de support (52) étant configurée et positionnée de sorte que le capteur (2) y disposé est positionné de la manette (50) d'au moins 5 mm.

5. Kit de rattrapage d'une pièce d'angle (34) comprenant une manette (50) et prévue comme support (32) pour une fraise de dentiste aux fins de mise à niveau pour l'utilisation dans un système (1) selon l'une des revendications 1 à 3, ayant un clip de fixation (62) montable sur un corps de base (60) de la pièce d'angle (34), où une canule de support (52) rigide est montée, à l'extrémité libre (54) de laquelle est disposé un capteur (2) associé au générateur de champ (4) et prévu pour une utilisation comme capteur de surveillance (30) de fraise et dont les conduites de signal (56) sont guidées dans le canal intérieur de la canule de support (52), la canule de support (52) étant configurée et positionnée de sorte que le capteur (2) y disposé est positionnable de la manette (50) d'au moins 5 mm.
